(19) Europäisches Patentamt
European Patent Office
Office européen des brevets

(11) Numéro de publication: **0 238 765 B1**

(12) # FASCICULE DE BREVET EUROPEEN

(45) Date de publication de fascicule du brevet: **21.07.93**
(51) Int. Cl.[5]: **A61K 31/135**

(21) Numéro de dépôt: **86402456.7**

(22) Date de dépôt: **04.11.86**

(54) **Utilisation du 2,2-Diphénoxy-N,N-diméthylaminoéthane pour l'amélioration de l'oxygénation cérébrale.**

(30) Priorité: **05.11.85 FR 8516359**

(43) Date de publication de la demande:
**30.09.87 Bulletin 87/40**

(45) Mention de la délivrance du brevet:
**21.07.93 Bulletin 93/29**

(84) Etats contractants désignés:
**AT BE CH DE ES FR GB GR IT LI LU NL SE**

(56) Documents cités:
**FR-A- 2 583 639**

**MEDITERR. MED., no. 155, mars 1978, pages 73-74; J. TASSY: "Utilisation de la médifoxamine en cardiologie"**

**R. BERKOW et al.: "The Merck Manual of Diagnosis and Therapy", édition 14, 1982, pages 488-494, Merck & Co.; "Myocardial ischemic disorders"**

**LE PROGRES MEDICAL, no. 104, 1976, pages 81-86; C. VERON: "A propos du Gerdaxyl-10 en médecine praticienne étude portant sur 102 malades"**

(73) Titulaire: **Albert ROLLAND S.A. Société dite**
**49, Rue St-André-des-Arts**
**F-75006 Paris(FR)**

(72) Inventeur: **Levy, Jean-Claude**
**45 Rue Commandant Mowat**
**F-94400 Vincennes(FR)**
Inventeur: **Bessin, Pierre**
**1 Allée Bosquet**
**F-91380 Chilly-Mozarin(FR)**
Inventeur: **Labaune, Jean-Pierre**
**10, rue Grands Cedres**
**F-77310 Moulignon par Ponthierry(FR)**

(74) Mandataire: **Burtin, Jean-François**
**Cabinet GEFIB, 59, rue Edouard-Vaillant**
**F-92300 Levallois-Perret (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

## Description

La présente invention concerne des compositions pharmaceutiques destinées à prévenir ou à corriger les effets d'une oxygénation insuffisante des cellules cérébrales.

On sait qu'un des effets marquants du vieillissement réside dans une diminution de la circulation cérébrale et d'un appauvrissement de l'apport aux cellules du cerveau de l'oxygène indispensable au maintien d'un métabolisme du glucose normal. Il en résulte une atrophie progressive de ces cellules et une déperdition importante en cellules fonctionnelles.

Dans ces conditions, il apparaît souhaitable de disposer d'un autre type de médicament qui permette de lutter contre les effets de la sénescence.

Le principe de ce type de médicament réside dans l'amélioration du fonctionnement de la cellule cérébrale, dans une plus grande résistance de celle-ci malgré un apport insuffisant d'oxygène, et dans l'accroissement du débit circulatoire cérébral. La cellule cérébrale, grâce à ce nouveau type de médicament, est en mesure de mieux résister à l'hypoxie ou de maintenir un fonctionnement normal même si l'apport d'oxygène vient à baisser dans des proportions importantes.

L'invention concerne donc l'utilisation du 2,2-diphénoxy-1-méthylaminoéthaneou d'un de ses sels d'addition avec un acide minéral ou organique, en vue de la réalisation d'un médicament destiné à améliorer l'oxygénation cérébrale.

Cet effet obtenu avec le médicament selon l'invention à base de 2,2-diphénoxy-N-diméthylaminoéthane ou Médifoxamine est d'autant plus surprenant que des substances comme la Nomifensine ou l'Amineptine qui exercent, au même titre que la Medifoxamine, un impact sur le système dopaminergique, sont dépourvues de telles propriétés.

Ce type de propriétés est retrouvé, de façon inconstante, d'une part chez un médicament à base d'extrait de Gingko biloba connu pour améliorer la circulation cérébrale, et d'autre part avec une spécialité pharmaceutique renfermant un analeptique respiratoire (almitrine) et une substance adrénolytique (la Raubasine).

Cet effet anti-anoxique ne semble donc pas pouvoir être rattaché à une propriété pharmacodynamique déjà connue.

Spécifiquement, l'invention se rapporte à l'utilisation du 2,2-bis-phénoxy-N,N-diméthylaminoéthane ou Medifoxamine,ou d'un de ses sels d'addition avec un acide minéral ou organique en vue de la réalisation d'un médicament destiné à améliorer l'oxygénation cérébrale.

La Medifoxamine est une substance connue depuis longtemps, notamment sous forme pharmaceutique (Mediterr. Med. N° 155 (Mars 1978) p. 73 - 74), mais dont l'étude a été reprise récemment.Sur le plan chimique, il s'agit d'un composé original qui n'est apparenté à aucune famille de psychotropes connus.

Des compositions pharmaceutiques, à base de Medifoxamine, ont été décrites par J. Tassy - Mediterr. Med. N° 155 (Mars 1978) p. 73 - 74, pour le traitement de certains troubles cardiaques.

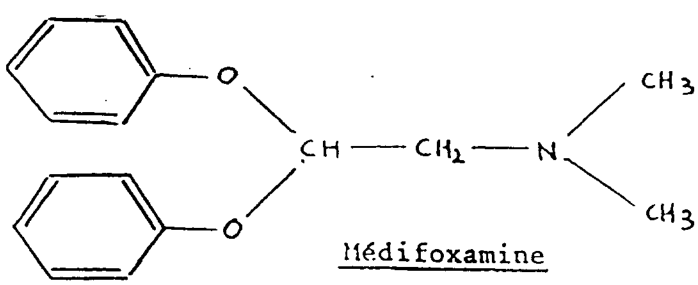

Médifoxamine

Des compositions pharmaceutiques, à base de Medifoxamine, utiles dans le traitement des symptômes somatiques liés à un état psychotique (troubles digestifs, colopathies, hypertension, tachycardies, algies diverses) ont été également décrites par C. VERON Progrès Med. 104 (1976) 81- 86.

Une mise au point des propriétés pharmacologiques du produit a été publiée par J. DELAUNAY et Coll. (Ann. Med. Psychol. (1982) T.140 P. 1: Des études pharmacologiques ont été effectuées par Hackct, par Lapras et par Simon (rapports d'expertise Amphar-Rolland). Les différents tests pharmacologiques ont montré : une activité anxiolytique mise en évidence sur les tests de comportement, une action antidépressive comparable à celle de l'imipramine et de la maprotiline, une activité antisérotonine, une action inductrice du sommeil barbiturique, l'absence d'effet adrénergique, une activité anticholinergique très réduite. L'étude de l'effet de l'alcool associé à la médifoxamine lors d'intoxication aiguë ou lors d'administration prolongée subtoxique n'a pas permis de révéler la potentialisation par la médifoxamine. La DL 50 chez la souris et le

rat sont respectivement de 750 et 1000 mg/kg. Les propriétés pharmacologiques étudiées comparativement aux produits de référence laissaient prévoir chez l'homme une posologie utile de 3 mg/kg.

La publication J. Tassy (Mediterr. Med. N° 155 (Mars 1978) p. 73) mentionne que la tolérance de la Médifoxamine est excellente et que seul un cas de somnolence diurne, avec insomnie nocturne, a été observé pour 21 patients.

Sur le plan biologique, on ne relève aucune anomalie chez les patients chez lesquels un contrôle biologique a pu être pratiqué avant et à l'issue de la période d'observation.

Par ailleurs, chez les patients qui ont pu être contrôlés 3 à 6 mois après la fin de l'étude, on n'observe pas non plus de modification des paramètres biologiques.

Les médicaments, selon l'invention, renferment une quantité neurologiquement active de Medifoxamine comprise entre 50 et 750mg de principe actif ou d'une quantité équivalente d'un de ses sels.

La quantité de principe actif peut varier en fonction de la voie d'administration et de la forme physique du principe actif. Si le principe actif est sous une forme physique soluble dans l'eau ou dispersible dans les milieux physiologiques, la teneur en principe actif par unité de prise sera en général de 50 à 400 mg. Si le principe actif est sous forme insoluble ou non dispersible dans les milieux aqueux, la teneur en principe actif sera plus élevée et en général variant de 250 à 750 mg par prise unitaire.

En outre, en fonction de la voie d'administration, les concentrations requises peuvent être plus faibles ou plus élevées, étant entendu que pour la voie digestive, les quantités de Medifoxamine sont plus importantes que par voie parentérale.

Les médicaments, selon l'invention, sont ceux qui conviennent pour l'administration par voie parentérale, orale, rectale ou percutanée. On citera, en particulier, les comprimés nus ou enrobés, les dragées, les gélules, les capsules molles, les pilules, les micro-granules, les gouttes, les solutions ou suspensions buvables, les solutés injectables, les suppositoires et les solutions pour usage transcutané. Les formes préférées sont les comprimés pelliculés, les gélules ou les dragées.

Les excipients ou les véhicules qui conviennent pour les médicaments, selon l'invention, sont l'eau ou les liquides aqueux salins, les amidons, la cellulose microcristalline comme l'Avicel (R), le phosphate di- ou tricalcique, le phosphate de magnésium, le sulfate de calcium, le lactose, la silice colloïdale, les alcoylcelluloses, le stéarate de magnésium, le talc, les carboxyméthylcelluloses comme l'Acdisol (R), les carboxyméthylamidons, les polyvinylpyrrolidones solides ou liquides à température ordinaire, les polyéthyléneglycols solides ou liquides, les stéarates de polyéthylèneglycol ou le beurre de Cacao.

La posologie journalière peut varier dans des limites sensibles en fonction du poids du sujet, de l'indication thérapeutique et de l'ancienneté de la maladie à traiter. La posologie journalière peut, de ce fait, s'échelonner de 150 à 800 mg répartie en une à trois prises dans la journée.

L'invention comprend également un procédé d'obtention des compositions pharmaceutiques améliorant l'oxygénation cérébrale, qui consiste en ce qu'on incorpore ou on ajoute à une quantité neurologiquement active du 2,2-diphénoxy-N,N-diméthylaminoéthane ou la quantité équivalente d'un de ses sels d'addition avec un acide minéral ou organique, un ou plusieurs excipients ou véhicules inertes non toxiques, pharmaceutiquement acceptables.

Les exemples suivants illustrent l'invention sans toutefois la limiter.

EXEMPLE I.

Comprimés sécables renfermant 100 mg de fumarate de 2,2-diphénoxy-N,N-diméthylaminoéthane :

| | |
|---|---|
| . fumarate de 2,2-diphénoxy-N,N-diméthylaminoéthane | 100 g |
| . phosphate dicalcique | 140 g |
| . cellulose microcristalline | 60 g |
| . amidon de maïs | 16 g |
| . stéarate de magnésium | 4 g |

pour 1.000 comprimés sécables terminés à un poids moyen de 0,320g.

EXEMPLE II

Etude pharmacologique des compositions selon l'invention :

A. - Activité de la Médifoxamine sur le métabolisme cérébral chez le chien anesthésié.

L'étude a été réalisée sur des lots de 4 chiens anesthésiés au mébubarbital, à la dose de 30mg/kg I.V. Les dosages biochimiques sont effectués au niveau de l'artère vertébrale et de la veine jugulaire. Les mesures de débit de l'artère vertébrale et le contrôle de la $PO_2$ artérielle et de la consommation d'oxygène cérébrale ont été faits avant et après l'injection de Médifoxamine à la dose de 10 mg/kg.

Les mesures retenues ont été celles des pics d'activité pour les différents paramètres considérés.

Résultats

L'injection intraveineuse de médifoxamine à la dose de 10 mg/kg a trois conséquences essentielles:

1) Augmentation de la $PO_2$ artérielle, qui passe de:
   71 ± 8 à 91,4 ± 15 mm Hg
2) Augmentation du débit de l'artère vertébrale qui passe de:
   85 ± 26 à 210 ± 92 ml/mn
3) Tendance à l'augmentation de la consommation d'oxygène.

| $PO_2$ (pic maxi) mmHg | | Débit (Pic maxi) ml/mn | | Consomm. $O^2$ (Pic maxi) vol/mn | |
|---|---|---|---|---|---|
| Avant | Après | Avant | Après | Avant | Après |
| 72 | 90 | 50 | 325 | 0,608 | 1,781 |
| 80 | 116 | 120 | 225 | 0,4 | 7,4 |
| 72 | 78 | 80 | 175 | 1,24 | 0,30 |
| 58 | 81 | 75 | 75 | 0,67 | 1,92 |
| 73 | 92 | 100 | 250 | - | - |
| 71 ± 8 | 91,4 ± 15 | 85 ± 26 | 210 ± 92 | 0,73 ± 0,36 | 2,85 ± 3,41 |
| $\alpha < 0,05$ | | $\alpha < 0,05$ | | NS | |

En conclusion, ces résultats vont dans le sens d'une possible action bénéfique de la Médifoxamine dans l'hypoxie cérébrale.

B.- Recherche d'un effet protecteur de la Médifoxamine contre l'hypoxie hypobare chez le rat mâle

Les produits essayés sont administrés par voie intrapéritonéale ou par voie orale 30 mm ou 60 mn avant la mise en caisson hypobare des rats. La pression à l'intérieur du caisson est de 180 mm Hg.

On détermine le temps de survie avant arrêt de la respiration.

1. Administration des produits par voie intrapéritonéale

La Médifoxamine, aux doses de 50 et 100 mg/kg, et un extrait de Gingko biloba à la dose de 200 mg/kg, augmentent le temps de survie des animaux, de manière significative.

Une composition pharmaceutique à base d'Almitrine et de Raubasine à la dose de 50 mg/kg prolonge ce temps, sans que la différence, avec les témoins, apparaisse significative.

La Nomifensine (50 mg/kg) n'a aucun effet.

2. Administration des produits par voie orale

La Médifoxamine (100 mg/kg) et une composition pharmaceutique à base d'Almitrine et de Raubasine (100 mg/kg) augmentent le temps de survie de manière significative.

L'extrait de Gingko biloba à la dose de 300 mg/kg prolonge ce temps; la différence, avec les témoins, n'est pas significative.

La Nomifensine (50 mg/kg) et l'Amineptine (100 mg/kg) ne présentent aucune activité.

Les tableaux suivants rassemblent les résultats obtenus.

RECHERCHE D'UN EFFET PROTECTEUR DE LA MEDIFOXAMINE CONTRE L'HYPOXIE HYPOBARE CHEZ LE RAT MÂLE

APRES ADMINISTRATION INTRA-PERITONEALE

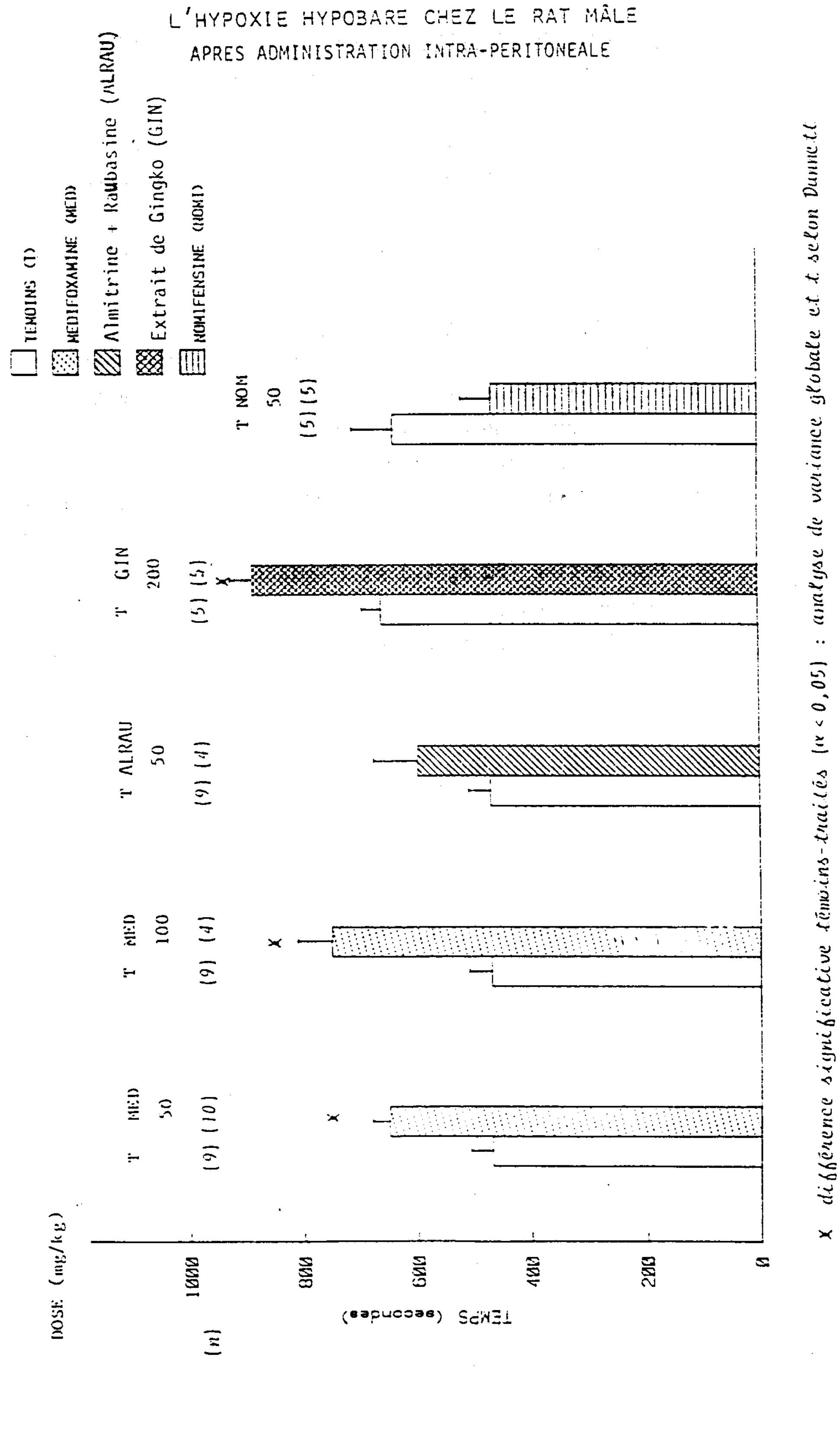

RECHERCHE D'UN EFFET PROTECTEUR DE LA MEDIFOXAMINE
CONTRE L'HYPOXIE HYPOBARE CHEZ LE RAT MALE
APRES ADMINISTRATION PAR VOIE ORALE

| TEMOINS<br>CMC 1% 1 ml/100 g<br>survie en seconde | MEDIFOXAMINE<br>100 mg/kg<br>CMC 1% 1 ml/100 g<br>P.O<br>survie en seconde | EXTRAIT DE GINCKO<br>300 mg/kg<br>CMC 1% 1 ml/100 g<br>P.O<br>survie en seconde | NOMIFENSINE<br>50 mg/kg<br>CMC 1% 1 ml/100 g<br>P.O<br>survie en seconde |
|---|---|---|---|
| 805 | 810 | 550 | 375 |
| 180 | 850 | 690 | 770 |
| 910 | 920 | 750 | 495 |
| 435 | 1035 | 890 | 590 |
| 730 | 1010 | 570 | 240 |
| 630 | 770 | 550 | 510 |
| 615 | 550 | 810 | 525 |
| 858 | 765 | 735 | 440 |
| 405 | 705 | 750 | 270 |
| 660 | 540 | 510 | 570 |
| 630 | 750 | | |
| 500 | 645 | | |
| 615 | 620 | | |
| 525 | 690 | | |
| 610 | 930 | | |
| 665 | 790 | | |
| 570 | 570 | | |
| 540 | 450 | | |
| 525 | 570 | | |
| 465 | - | | |
| 594 ± 37 | 735 ± 38 | 680 ± 41 | 479 ± 50 |
| Test Bartlett<br>$X_3^2$ = 0,80 NS<br>t de Dunnett<br>$F_{55}^3$ = 6,51 "S" | $t_{55}$ = 2,79 "S" | $t_{55}$ = 1,42 "N.S" | $t_{55}$ = 1,88 "N.S" |
| | $0{,}01 < \alpha < 0{,}05$ | | |

## Revendications

**1.** L'utilisation du 2,2-diphenoxy NN-diméthyl amino éthane ou d'un de ses sels d'addition avec un acide minéral ou organique pour la fabrication d'un médicament destiné à améliorer l'oxygénation cérébrale.

2. Utilisation selon la revendication 1 dans laquelle le 2,2-diphénoxy NN-diméthylamino éthane est utiltsé à une dose neurologiquement active.

3. Utilisation selon la revendication 1 ou 2 dans laquelle la dose neurologiquement active de 2,2-diphénoxy éthane ou d'un de ses sels s'échelonne de 50 à 750 mg par prise unitaire.

**Claims**

1. Utilization of 2,2-diphenoxy N,N-dimethylaminoethane or an addition salt thereof with a mineral or organic acid for the production of a drug intended for the improvement of brain oxygenation.

2. Utilization according to claim 1 wherein 2,2-diphenoxy N,N-dimethylaminoethane is used at a neurologically-active dosage.

3. Utilization according to claim 1 or claim 2 wherein the neurologically-active dosage of 2,2-diphenoxy N,N-dimethylaminoethane or one of its salts ranges from 50 to 750 mg per unit dosage.

**Patentansprüche**

1. Verwendung von 2,2-Diphenoxy N,N-dimethylaminoäthan oder einer seine Addition Salze mit einer anorganischen oder organivchen Säure, fur die Herstellung eines Arzneimittels bestimmt für die Verbesserung des Hirnssauerstoff durchfluss.

2. Verwendung nach Anspruch 1, worin 2,2-Diphenoxy N,N-dimethylaminoäthan an einer neurologischwirksame Dosis gebraucht wird.

3. Verwendung nach Ansprüche 1 oder 2, worin die neurologisch wirksame Dosis von 2,2-Diphenoxy N,N-dimethylaminoäthan oder einer seiner Salze von 50 bis 750 mg pro Einzeldosis sich erstreckt.